(19) Europäisches Patentamt
European Patent Office
Office européen des brevets

(11) Publication number: 0 152 229 B1

# (12) EUROPEAN PATENT SPECIFICATION

(45) Date of publication of patent specification: **22.04.92**

(51) Int. Cl.[5]: **C07D 493/08**, A01N 43/90, //(C07D493/08,319:00,319:00)

(21) Application number: **85300619.5**

(22) Date of filing: **30.01.85**

The file contains technical information submitted after the application was filed and not included in this specification

## (54) A class of pesticides comprising 1,4-bis-substituted-2,6,7-trioxabicyclo(2.2.2)octanes.

(30) Priority: **30.01.84 US 575843**
**23.01.85 US 692818**

(43) Date of publication of application:
**21.08.85 Bulletin 85/34**

(45) Publication of the grant of the patent:
**22.04.92 Bulletin 92/17**

(84) Designated Contracting States:
**BE CH DE FR GB IT LI NL SE**

(56) References cited:

**TETRAHEDRON LETTERS, vol. 24, no. 45, 1983; E.J.COREY et al.:"A new general synthetic route to bridged carboxylic ortho esters", pp. 5571-5574**

**JOURNAL OF THE AMERICAN CHEMICAL SOCIETY, vol. 92, 1970; R.D.BERTRAND et al.: "Unusual behavior of hexafluorobenzene and benzene in the aromatic nuclear magnetic resonance shift effect", pp. 2702-2709**

**THE JOURNAL OF THE AMERICAN CHEMICAL SOCIETY, vol. 76, 1954; O.C.DERMER et al.:"Extensions of the tollens condensation", pp. 1697-1699**

**TOXICOLOGY AND APPLIED PHARMACOLOGY, vol. 47, 1971; pp. 287-293**

**TOXICOLOGY AND APPLIED PHARMACOLOGY, vol. 36, 1976; pp. 261-279**

(73) Proprietor: **THE REGENTS OF THE UNIVERSITY OF CALIFORNIA**
**2199 Addison Street**
**Berkeley, California 94720(US)**

(72) Inventor: **Casida, John E.**
**1570 La Vereda Road**
**Berkeley, CA 94708(US)**
Inventor: **Palmer, Christopher J.**
**37 Clapgate Lane**
**Ipswich, Suffolk IP3 0RB(GB)**

(74) Representative: **Rollins, Anthony John**
**Group Patents & Agreements The Wellcome Foundation Ltd Langley Court**
**Beckenham Kent BR3 3BS(GB)**

EP 0 152 229 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new class of pesticides which are 1,4-bis-substituted-2,6,7-trioxabicyclo(2.2.2) octanes.

Pesticides are chemicals which combat the attacks of various pests on crops, livestock, man and their environment. They include insectides, fungicides, herbicides (or weed killers), nematicides, molluscicides, acaricides and parasiticides.

Many classes of compounds are known to exhibit pesticidal activity. Unfortunately, known pesticidal compositions may become less effective with time because of the development of resistance in the species against which they are used. Thus, there is a constant need for new types of pesticides.

An ideal pesticide has high effectiveness in controlling pests, and is biodegradable.

The present invention is concerned with providing pesticidal compositions having desirable properties such as those set forth above.

### Disclosure of the Invention

In accordance with an embodiment of the present invention a compound is provided having the formula R-C($CH_2O$)$_3$C-X wherein:

R and X are each organic substituents, having a pesticidal $LD_{50}$ value, expressed as micrograms of compound per gram of body weight of pest, of no more than 400, the $LD_{50}$ being determined by applying 0.5 microlitres of a solution of the compound in acetone or tetrahydrofuran topically to the ventrum of the abdomen of an anaesthetised adult female housefly (Musca domestica L), SCR strain, 3 to 5 days after emergence and determining mortality 24 hours after application of the test compound, the flies being maintained at 25° C.

In a preferred embodiment R is propyl, butyl, $C_{3-10}$ cycloalkyl or phenyl and X is $C_{6-10}$ cycloalkyl, $C_{6-10}$ cycloalkenyl, alkynyl or phenyl substituted by one or more halo, cyano, azido, nitro or $C_{1-2}$ alkyl, the alkyl being substituted by 1 to 3 halogen atoms, provided that R is not phenyl when X is 4-fluorophenyl.

In accordance with another embodiment of the present invention a compound is provided having the formula R-C($CH_2O$)$_3$C-X wherein R is propyl, butyl, $C_{5-6}$ cycloalkyl or phenyl and X is cyclohexyl, cycloheptyl, cyclohexenyl, ethynyl or phenyl substituted by halo, cyano, azido, nitro or $C_{1-2}$ alkyl, the alkyl being substituted by 1 to 3 halogen atoms, provided that R is not phenyl when X is 4-fluorophenyl.

Preferred compounds of formula R-C($CH_2O$)$_3$C−X are those wherein R is n-propyl, butyl, cyclopentyl or cylohexyl and X is cyclohexyl, cycloheptyl or phenyl substituted in the 3- and/or 4- position(s) by substituents independently selected from halo, cyano, azido and nitro, or wherein, R is phenyl and X is phenyl substituted in the 3- and/or 4- position(s) by a substituent independently selected from chloro and bromo.

More preferred compounds of formula R-C($CH_2O$)$_3$C-X are those wherein

R = n-propyl, iso-propyl, n-butyl, s-butyl, tert-butyl, cyclopentyl, cyclohexyl or phenyl; and wherein

X = ethynyl, cyclohexyl, cycloheptyl, cyclohex-3-enyl, 2-bicyclo[2.2.1]heptyl, 3-chlorophenyl, 4-chlorophenyl, 2-fluorophenyl, 4-fluorophenyl, 4-bromophenyl, 3,4-dichlorophenyl, 4-trifluoromethylphenyl, 4-azidophenyl, 4-nitrophenyl, 4-cyanophenyl, or 2,3,4,5,6-pentafluorophenyl, or wherein

X is 4-chlorophenyl and R is n-propyl, i-propyl, n-butyl, s-butyl, t-butyl, cyclopentyl, cyclohexyl, or phenyl; or wherein

X is cyclohexyl and R is cyclohexyl or phenyl; or wherein

R is cyclohexyl and X is phenyl, 4-fluorophenyl, 4-bromophenyl, 4-cyanophenyl, 3,4-dichlorophenyl, or cycloheptyl; or wherein

R is t-butyl and X is 2-fluorophenyl, 3-chlorophenyl, 4-fluorophenyl, 4-bromophenyl, 4-trifluoromethylphenyl, 4-nitrophenyl, 4-cyanophenyl, 4-azidophenyl, 3,4-dichlorophenyl, 2,3,4,5,6-pentafluorophenyl, n-pentyl, cyclohexyl, cycloheptyl, or ethynyl; or wherein

R is i-propyl and X is 4-nitrophenyl, cyclohexyl or cycloheptyl; or wherein

R is n-propyl and X is 2-bicyclo[2.2.1]heptyl or cyclohex-3-enyl.

In accordance with other embodiments of the present invention, pesticidal compositions comprising a compound as previously defined are provided which are used to kill selected insects.

The preferred pesticidal compositions of the present invention have acceptably high pesticidal activity, particularly when used with a synergist, and are biodegradable. They have been shown to exhibit significant pesticidal activity against such diverse insects as the common housefly, American and German cockroaches, aphid, milkweed bug and mosquito larva.

Best Mode for Carrying Out the Invention

In accordance with the present invention, a compound is provided having the formula R-C($CH_2O$)$_3$C-Xwherein R and X are each organic substituents as defined above at the 4- and 1- positions respectively in the compound. It has been shown that this class of compounds includes a number of chemicals which exhibit significant pesticidal activity. Tables 1 and 2, to which further reference is made later, show the relative effectiveness for control of houseflies and American cockroaches of a number of compounds of the general formula set forth above, both alone and with the synergist piperonyl butoxide (PB). The abbreviations used are as follows: Pr-propyl, Bu-butyl, Pen-pentyl, Hex-hexyl, Hept-heptyl, Ph-phenyl, n-normal, i-iso, s-secondary, t-tertiary, and c-cyclo.

The pesticides of the present invention exhibit a pesticidal activity, as $LD_{50}$ in micrograms of the pesticide per gram of body weight of a selected pest, of no more than about 400. For example, such an activity has been shown as an insecticide against Musca domestica as is discussed below. The pesticides of the present invention alternatively or additionally exhibit a pesticidal activity, as $LD_{50}$ in micrograms of the pesticide per gram of body weight of a selected pest, of no more than about 60 and more preferably of no more than about 20. Such an activity has been shown as an insecticide against Musca domestica as is discussed below.

The use of synergists with pesticides is well known and is discussed in detail in the publication "Mixed-Function Oxidase Involvement in the Biochemistry of Insecticide Synergists", J. E. Casida, J. Agric. Food Chem., 18, 753-772 (1970). The compounds of the present invention become even more effective with synergists which function to inhibit microsomal cytochrome P-450 oxidases that detoxify the pesticide thereby allowing a longer period for pesticidal action and consequently higher toxicity. Such synergists include those listed in the above publication plus other synergists which function in the manner stated.

The compositions of the present invention may be used in combination with an inert carrier that serves as a diluent or vehicle for the active pesticides. For example, the toxicant may be dissolved in petroleum hydrocarbons, tetrahydrofuran, acetone, cellosolves or any other suitable inert carrier prior to use. Alternatively, the toxicant may be adsorbed on a solid inert carrier such as talc, clay, finely ground silica or the like.

Contacting of pests with the pesticides of the present invention can be by any effective and convenient method, including any of the various methods of contacting well known in the art and used for delivering prior art pesticides to insects or other pests. For example, the pesticide may be utilized as a spray, an aerosol, dust or granules, or may be impregnated into or coated onto a structure with which the insect or other pests may come into contact, may be mixed with or impregnated into bait, may be incorporated with a substance or structure which slowly releases it in an area normally frequented by the pest or may be incorporated with and/or into other formulations which are directed into contact with the pest or placed where the pest will be likely to contact such formulations. The pesticides may be mixed with an inert carrier to facilitate delivery of the pesticides to the pests.

The toxicity to houseflies is dependent on both the R and X substituents as shown in Table 1 giving the first definitive evidence of effectiveness and further refined evaluations. The 1,4-bis-substituted-2,6,7-trioxabicyclo[2.2.2]octanes were tested both alone and along with PB.

The toxicity to American cockroaches of topically-applied 1,4-bis-substituted-2,6,7-trioxabicyclo[2.2.2]-octanes was tested both alone and along with PB. Table 2 presents the results of the testing.

Table 1. Housefly Control by 1,4-bis-Substituted-2,6,7-trioxabicyclo[2.2.2]octanes Alone and With the Synergist Piperonyl Butoxide

| $R-C(CH_2O)_3C-X$ R | X | Compd No.[a] | $LD_{50}$ μg/g, with PB (and alone) |
|---|---|---|---|
| n-Pr | 4-ClPh | 2 | 2.5(23)[2(10)][b] |
| i-Pr | 4-ClPh | 3 | 8.3(140)[12(100)] |
| n-Bu | 4-ClPh | 4 | 3.5(17)[4(23)] |
| s-Bu | 4-ClPh | 5 | 2.7(58) |
| t-Bu | 4-ClPh | 6 | 1.5(10)[1(15)] |
| c-Pen | 4-ClPh | 7 | 2.0(21) |
| c-Hex | 4-ClPh | 8 | 0.53(10)[0.4(37)] |
| Ph | 4-ClPh | 9 | 2.5(41)[7(400)]* |
| c-Hex | c-Hex | 13 | 0.63(8.5)[1(13)] |
| Ph | c-Hex | 14 | 7.0(375)[5(225)] |
| t-Bu | 2-FPh | 19 | 30(>500)[25(>500)] |
| t-Bu | 3-ClPh | 21 | 6.3(375)[4(125)] |
| t-Bu | 4-FPh | 23 | 5.5(>500)[3(>500)] |
| c-Hex | 4-FPh | 24 | 1.9(>500)[2(>500)] |
| t-Bu | 4-BrPh | 25 | 0.83(3.5)[0.4(4)] |
| c-Hex | 4-BrPh | 26 | 0.25(6.5) |
| t-Bu | $4-CF_3Ph$ | 27 | 53(>500) |
| i-Pr | $4-NO_2Ph$ | 28 | 11(>500) |
| t-Bu | $4-NO_2Ph$ | 29 | 5.0(>500) |
| t-Bu | 4-CNPh | 30 | 0.23(4.8) |
| c-Hex | 4-CNPh | 31 | 0.65(115) |
| t-Bu | $4-N_3Ph$ | 32 | 13(160) |
| t-Bu | $3,4-Cl_2Ph$ | 38 | 0.88(4.3)[1(5)] |

| | | | |
|---|---|---|---|
| *c*-Hex | 3,4-$Cl_2$Ph | 39 | 2.5(30) |
| *t*-Bu | 2,3,4,5,6-$F_5$Ph | 43 | 18(240) |
| *t*-Bu | *n*-Bu | 51 | 55(450)[50(450)] |
| *t*-Bu | *n*-Pen | 53 | 33(365) |
| *i*-Pr | *c*-Hex | 59 | 14(>500)[8(>500)] |
| *t*-Bu | *c*-Hex | 60 | 3.5(165)[2(40)] |
| *i*-Pr | *c*-Hept | 61 | 8.5(300) |
| *t*-Bu | *c*-Hept | 62 | 2.0(44) |
| *t*-Bu | ethynyl | 65 | 90(175)[125(150)] |
| *n*-Pr | 2-bicyclo[2.2.1]heptyl | 71 | 10(160) |
| *n*-Pr | cyclohex-3-enyl | 72 | 19(110) |
| *c*-Hex | *c*-Hept | 74 | 2.0(13) |

* Value based on suspension in acetone with only partial solution.

[a] Compound numbers are arbitrarily assigned.

[b] Data without brackets are refined evaluations whereas those with brackets are the first definitive evidence of effectiveness.

Table 2. Toxicity to American Cockroaches of Topically-Applied 1,4-bis-Substituted-2,6,7-trioxabicyclo[2.2.2]octanes

| $R-C(CH_2O)_3C-X$ R | X | Compd. No. | $LD_{50}$ µg/g with PB |
|---|---|---|---|
| n-Pr | 4-ClPh | 2 | 2 |
| n-Bu | 4-ClPh | 4 | 3 |
| t-Bu | 4-ClPh | 6 | 1[a] |
| c-Pen | 4-ClPh | 7 | 2 |
| c-Hex | 4-ClPh | 8 | 1[a] |
| Ph | 4-ClPh | 9 | 7 |
| c-Hex | c-Hex | 13 | >8 |
| t-Bu | 4-BrPh | 25 | 1[a] |
| c-Hex | 4-BrPh | 26 | 1 |
| t-Bu | $3,4-Cl_2Ph$ | 38 | >10 |
| t-Bu | c-Hex | 60 | 2[a] |

[a] At least 20-fold synergism by PB.

The above data indicate that compounds of the formula $R-C(CH_2O)_3C-X$ are often effective pesticides. R or X may be alkyl, alkynyl, cycloalkyl or cycloalkenyl, each of which may be normal, branched or substituted, or may be aryl or substituted aryl. When R is a normal or branched alkyl, a cycloalkyl or aryl, useful compounds result. Preferably, the number of carbon atoms in R is three to ten. More preferably R is n-propyl, t-butyl, cyclohexyl, cyclopentyl, i-propyl, n-butyl, s-butyl or phenyl. X is preferably cycloalkyl or substituted cycloalkyl or cycloalkenyl having six to ten carbon atoms, normal alkyl, alkynyl, or substituted phenyl. Effective pesticides result with cyclohexyl and cycloheptyl substituents. Other useful substituents include n-pentyl, cyclohex-3-enyl and 2-bicyclo[2.2.1]heptyl. The substituents on the phenyl group, when the phenyl group is the group X, are preferably halogens, cyano, nitro or azido groups.

Procedures of Synthesis

Methods for making compounds of the general formula set out above can be found in the following publications: "Structure-Toxicity Relationships of 1-Substituted-4-alkyl-2,6,7-trioxabicyclo[2.2.2]octanes," D.S. Milbrath, J.L. Engel, J.G. Verkade and J.E. Casida, Toxicology and Applied Pharmacology, 47, 287-293 (1979); "Structure-Toxicity Relationships of 2,6,7-Trioxabicyclo[2.2.2]octanes and Related Compounds," J.E. Casida, M. Eto, A.D. Moscioni, J.L. Engel, D.S. Milbrath and J.G. Verkade, Toxicology and Applied Pharmacology, 36, 261-279 (1976); "Nuclear Magnetic Resonance in Polycyclic Compounds. II. Long-Range $H^1$-$H^1$ and $H^1$-$P^{31}$ Coupling in Some Adamantane and Bicyclo[2.2.2]octane Derivatives," E.J. Boros, K.J. Coskran, R.W. King and J.G. Verkade, JACS, 88, 1140-1143 (1966); "Unusual Behavior of Hexafluorobenzene and Benzene in the Aromatic Nuclear Magnetic Resonance Shift Effect," R.D. Bertrand, R.D. Compton and J.G. Verkade, JACS, 92, 2702-2709 (1970); and "A New General Synthetic Route to Bridged Carboxylic Ortho Esters," E.J. Corey and N. Raju, Tetrahedron Letters, 24, 5571-5574 (1983).

Intermediates for these reactions are described by "Ketene Acetals. XXXIV. Tetra- and Pentamethylene Ketene Acetals," S.M. McElvain and R.E. Starn, Jr., JACS, 77, 4571-4577 (1955); "Preparation of Trimethylolisobutane by Condensation of Isovaleraldehyde with Formaldehyde," M.M. Ketslakh, D.M.

Rudkovskii, and F.A. Eppel, Oksosintez. Poluchenie Metodom Oksosinteza Al'degidov, Spritov i Vtorichnykh Produktov na ikh Osnove, Vses. Nauchn.-Issled., Inst. Neftekhim Protsessov, 156-163 (1963); "Extensions of Tollens Condensation," O.C. Dermer and P.W. Solomon, JACS, 76, 1697-1699 (1954); and "Cyclic Ethers Made by Pyrolysis of Carbonate Esters". D.B. Pattison, JACS, 79, 3455-3456 (1957).

Two specific methods (A and B) were used to prepare the trioxabicyclooctanes (Table 3). Each procedure started from a triol synthesized from the corresponding substituted-acetaldehyde by hydroxymethylation and subsequent crossed-Cannizzaro reaction (Dermer and Solomon, 1954; Ketslakh et al., 1963). The reaction scheme was:

$$RCH_2CHO \xrightarrow[\text{NaOH aqueous}]{CH_2O} RC(CH_2OH)_3$$

Each trioxabicyclooctane gave appropriate proton nuclear magnetic resonance (nmr) and mass spectrometry (MS) characteristics (Table 3). Abbreviations used in nmr data are s = singlet, d = doublet, 2xd = two doublets, dd = doublet of doublets, t = triplet, 2xt = two triplets, q = quartet and m = multiplet.

Table 3. Characterization Data of 1,4-bis-Substituted-2,6,7-trioxabicyclo[2.2.2]octanes

| No. | X | R | Proc. of Syn.* | $^1$H nmr[a] δ | mp (°C) | $[M+1]^{+b}$ |
|---|---|---|---|---|---|---|
| 2 | 4-ClPh | n-Pr | A | 0.9[3H, t, $CH_3$], 1.2-1.4[4H, m, $CH_2CH_2$], 4.15[6H, s, $(CH_2O)_3$], 7.4[2H, d, aromatic], 7.6[2H, d, aromatic] | 129-131 | 269 |
| 3 | 4-ClPh | i-Pr | A | 0.95[6H, d, $(CH_3)_2C$], 1.6[1H, m, CH], 4.15[6H, s, $(CH_2O)_3$], 7.3[2H, d, aromatic], 7.5[2H, d, aromatic] | 114-116 | 269 |
| 4 | 4-ClPh | n-Bu | A | 0.95[3H, t, $CH_3$], 1.2-1.5[6H, m, $(CH_2)_3$], 4.15[6H, s, $(CH_2O)_3$], 7.4[2H, d, aromatic], 7.6[2H, d, aromatic] | 111-114 | 283 |
| 5 | 4-ClPh | s-Bu | A | 0.85[3H, d, $CH_3$], 0.9[3H, t, $CH_3$], 1.2-1.6[3H, m, $CH_2CH$], 4.1[6H, s, $(CH_2O)_3$], 7.3 and 7.5(4H, 2xd, aromatic] | 119-120 | |
| 6 | 4-ClPh | t-Bu | A | 0.9[9H, s, $(CH_3)_3C$], 4.1[6H, s, $(CH_2O)_3$], 7.3 and 7.5[4H, 2xd, aromatic] | 176-178 | 283 |
| 7 | 4-ClPh | c-Pen | A | 1.1-1.75[9H, m, $(CH_2)_4CH$], 4.15[6H, s, $(CH_2O)_3$], 7.3 and 7.5[4H, 2xd, aromatic] | 170-173 | |
| 8 | 4-ClPh | c-Hex | A | 0.9-2.0[11H, m, $(CH_2)_5CH$], 4.15[6H, s, $(CH_2O)_3$], 7.4 and 7.65[4H, 2xd, aromatic] | 215-218 | 309 |
| 9 | 4-ClPh | Ph | A | 4.55[6H, s, $(CH_2O)_3$], 7.2-7.9[9H, m, aro- | 239-240 | |

| | | | | | | |
|---|---|---|---|---|---|---|
| | | | | matic] | | |
| 13 | c-Hex | c-Hex | A | 1.8-2.0[22H, m, $(CH_2)_5CH$ and $(CH_2)_5CH$], 3.95[6H, s, $(CH_2O)_3$] | 240-242 | 281 |
| 14 | c-Hex | Ph | A | 0.9-1.9[11H, m, $(CH_2)_5CH$], 4.2[6H, s, $(CH_2O)_3$], 7.0-7.4[5H, m, aromatic] | 195-196 | 275 |
| 19 | 2-FPh | t-Bu | A | 0.9[9H, s, $(CH_3)_3C$], 4.15[6H, s, $(CH_2O)_3$], 7.0-7.7[4H, m, aromatic] | 143-145 | 267 |
| 21 | 3-ClPh | t-Bu | A | 0.9[9H, s, $(CH_3)_3C$], 4.15[6H, s, $(CH_2O)_3$], 7.2-7.6[4H, m, aromatic] | 164-166 | |
| 23 | 4-FPh | t-Bu | A | 0.9[9H, s, $(CH_3)_3C$], 4.2[6H, s, $(CH_2O)_3$], 7.2-8.0[4H, m, aromatic] | 148-149 | 267 |
| 24 | 4-FPh | c-Hex | A | 0.9-2.0[11H, m, $(CH_2)_5CH$], 4.15[6H, s, $(CH_2O)_3$], 6.9-7.8[4H, m, aromatic] | 225-228 | 293 |
| 25 | 4-BrPh | t-Bu | A | 0.9[9H, s, $(CH_3)_3C$), 4.15[6H, s, $(CH_2O)_3$], 7.45[4H, s, aromatic] | 196-198 | |
| 26 | 4-BrPh | c-Hex | A | 0.9-1.9[11H, m, $(CH_2)_5CH$], 4.15[6H, s, $(CH_2O)_3$], 7.5(4H, s, aromatic] | 223-225 | |
| 27 | 4-$CF_3$Ph | t-Bu | A | 0.9[9H, $(CH_3)_3C$], 4.2[6H, s, $(CH_2O)_3$], 7.7[4H, q, aromatic] | 209-212 | |
| 28 | 4-$NO_2$Ph | i-Pr | B | 0.9[6H, d, $(CH_3)_2C$], 1.6[1H, s, CH], 4.15[6H, s, $(CH_2O)_3$], 7.75 and 8.2[4H, 2xd, aromatic] | 163-166 | 280 |

**Table 3 cont.**

| | | | | | | |
|---|---|---|---|---|---|---|
| 29 | 4-$NO_2$Ph | t-Bu | B | 0.9[9H, s, $(CH_3)_3C$], 4.2[6H, s, $(CH_2O)_3$], 7.75[2H, d, aromatic], 8.2[2H, d, aromatic] | 222-223 | |
| 30 | 4-CNPh | t-Bu | B | 0.9[9H, s, $(CH_3)_3C$], 4.2[6H, s, $(CH_2O)_3$], 7.65[4H, q, aromatic] | 207-208 | 274 |
| 31 | 4-CNPh | c-Hex | B | 0.95-1.8[11H, m, $(CH_2)_5CH$], 4.1[6H, s, $(CH_2O)_3$], 7.55-7.75[4H, q, aromatic] | 239-241 | |
| 32 | 4-$N_3$Ph | t-Bu | B | 0.9[9H, s, $(CH_3)_3C$], 4.15[6H, s, $(CH_2O)_3$], 7.0 and 7.6[4H, 2xd, aromatic] | 122-124 | 290 |
| 38 | 3,4-$Cl_2$Ph | t-Bu | A | 0.9[9H, s, $(CH_3)_3C$], 4.1[6H, s, $(CH_2O)_3$], 7.35-7.7[3H, m, aromatic] | 170-172 | 317 |
| 39 | 3,4-$Cl_2$Ph | c-Hex | A | 0.9-1.8[11H, m, $(CH_2)_5CH$], 4.1[6H, s, $(CH_2O)_3$], 7.35-7.7[3H, m, aromatic] | 192-195 | |
| 43 | $F_5$Ph | t-Bu | B | 0.9[9H, s, $(CH_3)_3C$], 4.15[6H, s, $(CH_2O)_3$] | 163-165 | 339 |
| 51 | n-Bu | t-Bu | A | 0.9[9H, s, $(CH_3)_3C$], 0.9[3h, t, $CH_3$], 1.1-1.9[6H, m, $(CH_2)_3$], 4.0[6H, s, $(CH_2O)_3$] | 37-38 | 229 |
| 53 | n-Pen | t-Bu | B | 0.8[9H, s, $(CH_3)_3C$], 0.85[3H, t, $CH_3$], 1.1-1.7[8H, m, $(CH_2)_4$], 3.9[6H, s, $(CH_2O)_3$] | 29-30 | |
| 59 | c-Hex | i-Pr | A | 0.85[6H, d, $(CH_3)_2C$], 1.0-2.0[12H, m, $(CH_2)_5CH$ and CH], 4.0[6H, s, $(CH_2O)_3$] | 118-121 | 241 |
| 60 | c-Hex | t-Bu | A | 0.85[9H, s, $(CH_3)_3C$], 1.0-2.0[11H, m, $(CH_2)_5CH$], 4.0[6H, s, $(CH_2O)_3$] | 154-155 | 255 |
| 61 | c-Hept | i-Pr | B | 0.85[6H, d, $(CH_3)_2C$], 1.2-2.0[14H, m, $(CH_2)_6CH$ and CH], 3.9[6H, s, $(CH_2O)_3$] | 106-108 | |
| 62 | c-Hept | t-Bu | B | 0.85[9H, s, $(CH_3)_3C$], 1.2-1.9[13H, m, | 178-181 | |

Table 3 cont.

| | | | | | | |
|---|---|---|---|---|---|---|
| | | | | $(CH_2)_6CH]$, 3.95[6H, s, $(CH_2O)_3]$ | | |
| 65 | Ethynyl | t-Bu | A[c] | 0.85[9H, s, $(CH_3)_3C]$, 2.5[1H, s, $C\equiv CH]$, 4.05[6H, s, $(CH_2O)_3]$ | 195-197 | 197 |
| 71 | 2-Bicyclo-[2.2.1]heptyl | n-Pr | B | 0.9[3H, t, $CH_3]$, 1.05-2.3[15H, m, $6xCH_2$ and 3xCH], 3.9[6H, s, $(CH_2O)_3]$ | 80-82 | |
| 72 | Cyclohex-3-enyl | n-Pr | B | 0.9[3H, t, $CH_3]$, 1.1-1.2[4H, m, $CH_2CH_2]$, 1.7-2.2[6H, m, $(CH_2)_3]$, 3.9[6H, s, $(CH_2O)_3]$, 5.6[2H, m, CH=CH] | 55-57 | |
| 74 | c-Hept | c-Hex | B | 0.85-1.95[24H, m, $(CH_2)_6CH$ and $(CH_2)_5CH]$, 3.9[6H, s, $(CH_2O)_3]$ | 188-190 | |

* Procedures of synthesis as specified in the text.

a Spectra obtained at 90 or 300 MHz for samples dissolved in deuterochloroform. Integrations were consistent with structural assignments.

b Quasi-molecular ion obtained with chemical ionization at 230 eV with methane (0.8 Torr).

c Compound 65 was prepared from the corresponding 1-bromoethyl derivative (prepared using Method A) via the vinyl and 1,2-dibromoethyl derivatives by sequential dehydrobromination, bromination and dehydrobromination.

Procedure A. Acid-catalyzed Condensation of a Triol with an Orthocarboxylate (Boros et al., 1966; Bertrand et al., 1970). The equation for the reaction being:

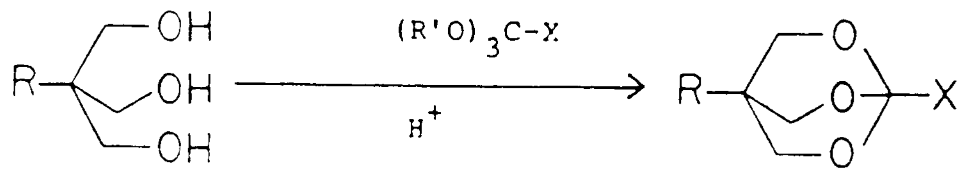


where R' may be alkyl or aryl, preferably methyl or ethyl. For example, a mixture of 2-t-butyl-2-hydroxymethyl-1.3-propanediol (R = t-Bu) (0.4g, 2.5mmol), trimethyl orthocyclohexanecarboxylate (X = c-Hex; R' = $CH_3$) (0.5g, 2.5mmol) and 4-toluenesulphonic acid (10mg) was heated to 160 °C until methanol distilled over. The residue was vacuum dried (at 1mm Hg) and then passed down a short basic alumina column to give trioxabicyclooctane 60 (X = c-Hex; R = t-Bu) (0.6g, 95%). Directly analogous procedures were used to prepare compounds 2-9, 13, 14, 19, 21, 23-27, 38, 39, 51, 59, 60 and 65.

Intermediate trimethyl orthocarboxylates were commercially available or were synthesized by either of two procedures illustrated with the methyl esters. In the first procedure, the appropriate benzotrichloride or benzotribromide (from bromination of the corresponding toluene with N-bromosuccinimide (NBS)) was subjected to halide displacement with methoxide (McElvain and Venerable, 1950). These methods were as follows:

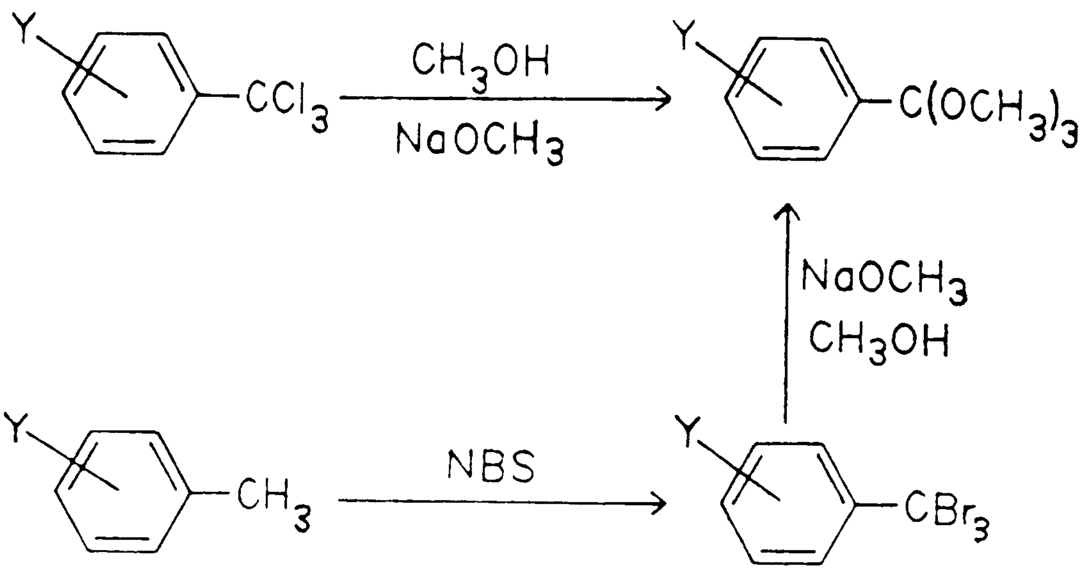


wherein Y represents hydrogen or one or more other groups such as halo or trifluoromethyl.

In the second procedure, the appropriate nitrile was treated with methanol and hydrochloric acid to obtain the imino ester hydrochloride and ultimately the trimethyl orthocarboxylate (McElvain and Starn, 1955). This procedure was as follows:

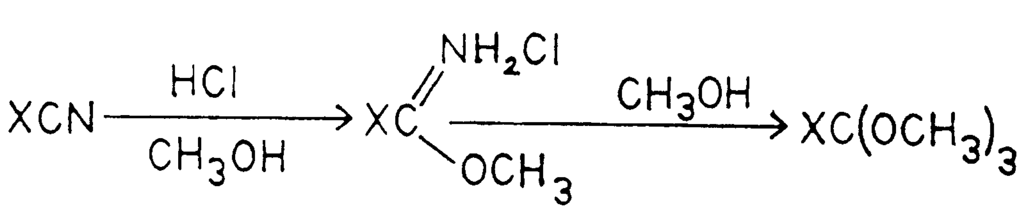


Procedure B. Rearrangement of an Acylated Hydroxymethyloxetane (Corey and Raju, 1983). Acylation of 3-substituted-3-hydroxymethyloxetanes (prepared from the appropriate trio] via pyrolysis of the carbonate ester) (Pattison, 1957) gives the corresponding oxetane esters which can be rearranged in the presence of

boron trifluoride etherate to form trioxabicyclooctanes. The equation for the reaction being:

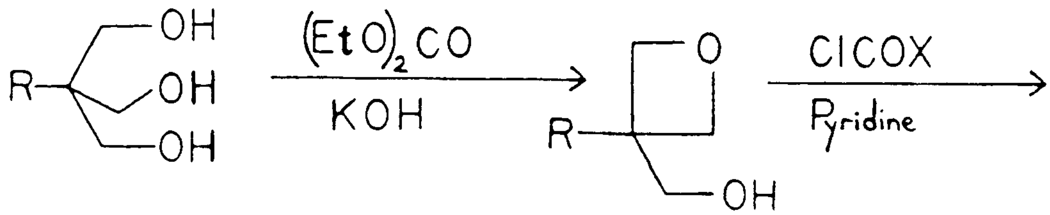


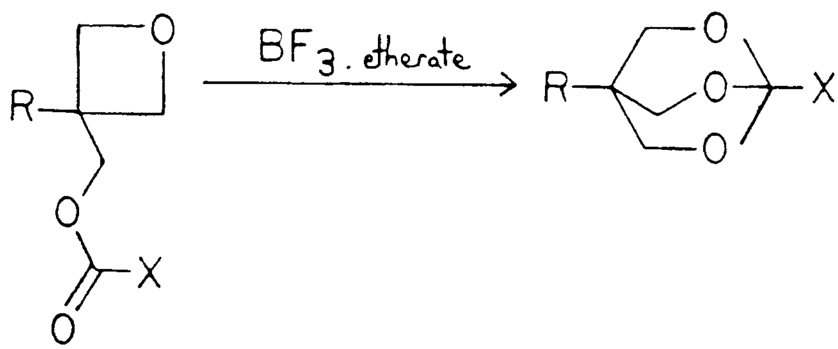


For example, 4-nitrobenzoyl chloride (2.28g, 12.3mmol) in dry dichloromethane (4ml) was added to 3-isopropyl-3-hydroxymethyloxetane (1.6g, 12.3mmol) in dry dichloromethane (15ml) and dry pyridine (2ml) at 0°C under a nitrogen atmosphere. The solution was stirred overnight, then extracted with water, dried (sodium sulfate), filtered and evaporated to leave the 4-nitrobenzoyl ester (3.kg, 99%) as a residue which was not purified farther. $^{1}H$ NMR ($CDCl_3$), $\delta$1.0 [6H, d, $(CH_3)_2C$], 2.3 [1H, m, C-CH], 4.55 [2H, s, $CH_2OCO$), 4.6 [4H, dd, $CH_2OCH_2$), 8.3 [4H, q, aromatic). This residue was dissolved in dry dichloromethane (15ml) under a nitrogen atmosphere, cooled to -55°C and boron trifluoride etherate (2ml) was added. The mixture was allowed to warm to room temperature and was then quenched with triethylamine, evaporated to dryness and partitioned between dichloromethane and water. The organic layer was separated, dried (potassium carbonate) and evaporated. The residue was purified by passage through a short basic alumina column to afford trioxabicyclooctane 28 (X = 4-$NO_2$Ph; R = i-Pr) (1.7g, 50%). Directly analogous procedures were used to prepare compounds 28-32, 43, 53, 61, 62, 71, 72 and 74.

Biological Activity Determination

Example 1: $LD_{50}$ for Musca domestica

The compounds listed in Table 1 were tested for insecticidal activity by dissolving them in acetone or in tetrahydrofuran if they were insoluble in acetone. Subsequent dilutions were prepared using the same solvent. The compound solutions (0.5 microliter) were applied topically to the ventrum of the abdomen of anesthetized adult female houseflies (Musca domestica L., SCR strain, 3-5 days after emergence, 20 mg each). The toxicity studies were varied by treating the houseflies topically with PB at 250 micrograms per gram, two to three hours prior to administering the toxicant. The treated houseflies were provided sugar and water, and mortality was determined after 24 hours at 25°C. The data in Table 1 are reported as the lethal dose, in micrograms of toxicant per gram of insect weight required to kill 50% of the fly population and are referred to as the $LD_{50}$ values.

Table 1 gives the structure-activity relationships for the test compounds on houseflies and on houseflies pre-treated with PB. Many of the compounds are active in the same potency range in this test as current commercial pesticides.

Significant insecticidal activity against the common housefly was found with suitable combinations of the following R and X substituents:

R = n-propyl, iso-propyl, n-butyl, s-butyl, tert-butyl, cyclopentyl, cyclohexyl, or phenyl; and

x = n-butyl, n-pentyl, cyclohexyl, cycloheptyl, 4-cyanophenyl, 3,4-dichlorophenyl, 3-chlorophenyl, 4-chlorophenyl, 4-bromophenyl, 4-fluorophenyl, 4-nitrophenyl, 4-azidophenyl, pentafluorophenyl, 2-bicyclo[2.2.1]heptyl, phenyl, 2-fluorophenyl, 4-trifluoromethylphenyl, ethynyl, or cyclohex-3-enyl.

Other X or R substituents which may confer activity are substituted-cycloalkanes or -cycloalkenes of 5 to 10 carbon atoms. R may also confer activity when it is a substituted-phenyl group. It is preferred that R is not phenyl when X is phenyl or 4-fluorophenyl.

Compounds having pesticidal activity include those wherein R is a normal or branched propyl or butyl, or phenyl or cycloalkyl having 3 to 10 carbon atoms and X is cycloalkyl or cycloalkenyl having 6 to 10 carbon atoms, or substituted phenyl. Another group of compounds with pesticidal activity are those where R is normal or branched propyl or butyl, phenyl or cycloalkyl having 5 or 6 carbon atoms and X is cyclohexyl, cycloheptyl, cyclohexenyl, ethynyl, or phenyl substituted by halo, cyano, azido, nitro or $C_{1-2}$ alkyl, the said $C_{1-2}$ alkyl being substituted by 1 to 3 halogen atoms. Yet another group of active compounds are those wherein R is n-propyl, normal or branched butyl, cyclopentyl or cyclohexyl and X is cyclohexyl, cycloheptyl or phenyl substituted in the 3- or 4- position by one or more of halo, cyano, azido, and nitro. Still another group of active compounds are those wherein R is phenyl and X is phenyl substituted in the 3- and/or 4-positions with chloro and/or bromo.

Compounds which have been prepared and tested for insecticidal activity and which exhibit very high activity (generally an $LD_{50}$ of less than or equal to about 5.5µg/g when used along with PB) include those wherein:

X is 4-chlorophenyl and R is n-propyl, n-butyl, s-butyl, t-butyl, cyclopentyl, cyclohexyl or phenyl;
R is t-butyl and X is cyclohexyl, cycloheptyl, 4-fluorophenyl, 4-bromophenyl, 4-nitrophenyl, 4-cyanophenyl or 3,4-dichlorophenyl; and
R is cyclohexyl and X is cyclohexyl, cycloheptyl, 4-fluorophenyl, 4-bromophenyl, 4-cyanophenyl or 3,4-dichlorophenyl.

On a more general basis, R and X can be substantially any organic substituents, so long as the required activity is found. To date, activity has been found where R and X are n-alkyl, branched-alkyl, cycloalkyl, substituted-cycloalkyl, phenyl, or substituted-phenyl substituents. While not all compounds with these substituents are active, many suitable combinations exist. Furthermore, simple tests as set out above with houseflies, or similar tests run with any desired pest, quickly determine which combinations are suitable for use against any particular pest.

Example 2: Tests with Other Insects

The compounds listed in Table 2 were tested for toxicity to adult male American cockroaches (Periplaneta americana) by applying test solutions, as in Example 1, to the thorax using 1.0 µl carrier solvent per insect. In each case the cockroaches were also tested after topical pretreatment with PB at 250 µg/g 2 hours before administering the trioxabicyclooctane. $LD_{50}$ values were established after 24 hours at 25 °C. The compounds tested are strongly synergized by PB to achieve a potency similar to that with PB-treated houseflies except for two compounds (13 and 38) which are >10-fold more toxic to houseflies than to cockroaches. Other tests showed that compound 6 was also toxic to the mosquito larva, black bean aphid, German cockroach and milkweed bug. Thus, the wide range of usefulness of the new class of pesticides set forth herein has been established.

Example 3: Tests with Other Synergists

Other synergists known to serve as inhibitors of oxidative detoxification can be used in place of PB. This has been confirmed with tests on houseflies with the compound wherein R is tert-butyl and X is 4-chlorophenyl. The synergism factor achieved with PB at 250 micrograms per gram is also achieved when this synergist is replaced with either O-(2-methylpropyl) O-(2-propynyl) phenylphosphonate at 125 micrograms per gram or O,O-diethyl O-phenyl phosphorothioate at 50 micrograms per gram. In these tests the synergist and insecticide were applied at the same time to more closely simulate actual use situations. These synergists are relatively low in cost and mammalian toxicity. Many compounds are therefore useful and of economic benefit as synergists in improving the insecticidal activity of insecticidal trioxabicyclooctanes.

Example 4: Test of Mammalian Toxicity

The mammalian toxicities of two of the compounds of the present invention were determined by

individually dissolving each compound in olive oil and administering each resulting solution intraperitoneally to mice for determination of the mortality 24 hours later. The compounds were: R is tert-butyl and X is cyclohexyl (60); R is isopropyl and X is cyclohexyl (59). The injected $LD_{50}$ values for the tert-butyl and isopropyl compounds are greater than 200 and 500 milligrams per kilogram body weight, respectively.

As is apparent, some compounds of the present invention have relatively low mammalian toxicity, specifically an injected $LD_{50}$ of greater than 200 milligrams of compound per kilogram of body weight. The mammalian toxicity may vary significantly depending upon the solvent carriers, routes of administration and species of mammal.

Industrial Applicability

The compounds of the present invention show significant activity as pesticides against widely different types of insects. The compounds biodegrade readily.

## Claims

1. A compound $R-C(CH_2O)_3C-X$ wherein R and X are each organic substituents, having a pesticidal $LD_{50}$ value, expressed as micrograms of compound per gram of body weight of pest, of no more than 400, the $LD_{50}$ being determined by applying 0.5 microlitres of a solution of the compound in acetone or tetrahydrofuran topically to the ventrum of the abdomen of an anaesthetised adult female housefly (Musca domestica L), SCR strain, 3 to 5 days after emergence and determining mortality 24 hours after application of the test compound, the flies being maintained at 25 °C.

2. A compound according to claim 1 wherein R is propyl, butyl, $C_{3-10}$ cycloalkyl or phenyl and X is $C_{6-10}$ cycloalkyl, $C_{6-10}$ cycloalkenyl, alkynyl or phenyl substituted by one or more halo, cyano, azido, nitro or $C_{1-2}$ alkyl, the alkyl being substituted by 1 to 3 halogen atoms, provided that R is not phenyl when X is 4-fluorophenyl.

3. A compound according to claim 2 wherein R is propyl, butyl, $C_{5-6}$ cycloalkyl or phenyl and X is cyclohexyl, cycloheptyl, cyclohexenyl, ethynyl or phenyl substituted by halo, cyano, azido, nitro or $C_{1-2}$ alkyl, the said $C_{1-2}$ alkyl being substituted by 1 to 3 halogen atoms, provided that R is not phenyl when X is 4-fluorophenyl.

4. A compound according to either claim 2 or 3 wherein R is n-propyl, butyl, cyclopentyl or cyclohexyl and X is cyclohexyl, cycloheptyl, or phenyl substituted in the 3- and or 4- position(s) by substituents independently selected from halo, cyano, azido and nitro.

5. A compound according to either claim 2 or 3 wherein R is phenyl and X is phenyl substituted in the 3- and or 4- position(s) by a substituent independently selected from chloro and bromo.

6. A compound according to claim 1 selected from 1-(4-chlorophenyl)-4-n-propyl-2,6,7-trioxabicyclo[2.2.2]-octane, 1-(4-chlorophenyl)-4-n-butyl-2,6,7-trioxabicyclo[2.2.2]octane, 1-(4-chlorophenyl)-4-s-butyl-2,6,7-trioxabicyclo[2.2.2]octane, 1-(4-chlorophenyl)-4-t-butyl-2,6,7-trioxabicyclo[2.2.2]octane, 1-(4-chlorophenyl)-4-cyclopentyl-2,6,7-trioxabicyclo[2.2.2]octane, 1-(4-chlorophenyl)-4-cyclohexyl-2,6,7-trioxabicyclo[2.2.2]octane, 1-(4-chlorophenyl)-4-phenyl-2,6,7-trioxabicyclo[2.2.2]octane, 1-cyclohexyl-4-t-butyl-2,6,7-trioxabicyclo[2.2.2]octane, 1-cycloheptyl4-t-butyl-2,6,7-trioxabicyclo[2.2.2]octane, 1-(4-fluorophenyl)-4-t-butyl-2,6,7-trioxabicyclo[2.2.2]octane, 1-(4-bromophenyl)-4-t-butyl-2,6,7-trioxabicyclo-[2.2.2]octane, 1-(4-nitrophenyl)-4-t-butyl-2,6,7-trioxabicyclo[2.2.2]octane, 1-(4-cyanophenyl)-4-t-butyl-2,6,7-trioxabicyclo[2.2.2]octane, 1-(3,4-dichlorcphenyl)-4-t-butyl-2,6,7-trioxabicyclo[2.2.2]octane, 1-cyclohexyl-4-cyclohexyl-2,6,7-trioxabicyclo[2.2.2]octane, 1-cycloheptyl-4-cyclohexyl-2,6,7-trioxabicyclo-[2.2.2]octane, 1-(4-fluorophenyl)-4-cyclohexyl-2,6,7-trioxabicyclo[2.2.2]octane, 1-(4-bromophenyl)-4-cyclohexyl-2,6,7-trioxablcyclo[2.2.2]octane, 1-(4-cyanophenyl)-4-cyclohexyl-2,6,7-trioxablcyclo[2.2.2]-octane, and 1-(3,4-dichlorophenyl)-4-cyclohexyl-2,6,7-trioxabicyclo[2.2.2]octane.

7. A pesticidal composition comprising a compound according to any one of claims 1 to 6 in admixture with an inert carrier.

8. A pesticidal composition according to claim 7 which further comprises a synergist which inhibits

oxidative detoxification of the pesticide.

9. A compound as defined in any one of claims 1 to 6 for the control of pests.

10. A method of controlling pests comprising contacting said pests or their environment with an effective amount for controlling said pests of a compound as defined in any one of claims 1 to 6.

11. A method of controlling insects according to claim 10 wherein said pests or their environment are also contacted with a synergist which inhibits oxidative detoxification of the pesticide.

12. A method of preparing a compound $R-C(CH_2O)_3C-X$ according to any one of claims 1 to 6:
a) from a triol $R-C(CH_2OH)_3$ which comprises acid catalysed condensation of the triol with an orthocarboxylate $(R'O)_3C-X$ or
b) by the rearrangement of an acylated hydroxymethyloxetane

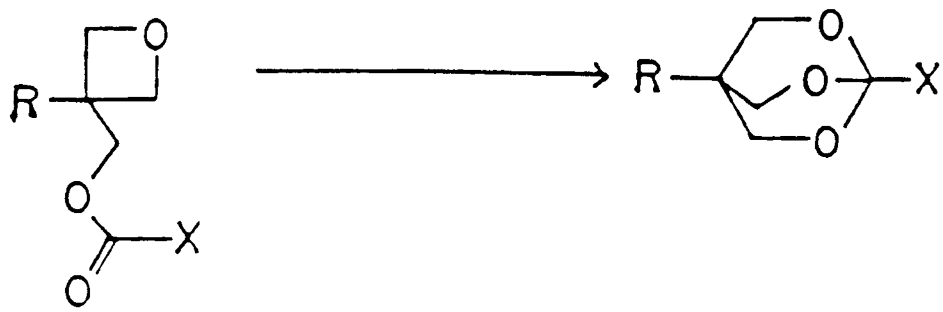

wherein R and Y are as defined in relation to any one of claims 1 to 6.

## Revendications

1. Composé de formule :

$R-C(CH_2O)_3C-X$

où R et X sont chacun un substituant organique, ayant une $DL_{50}$ pesticide, exprimée en µm de composé par g de poids du corps de l'organisme nuisible, n'excédant pas 400, la $DL_{50}$ étant déterminée par application de 0.5 µl d'une solution du composé dans l'acétone ou le tétrahydrofuranne topiquement sur la face ventrale de l'abdomen de mouches domestiques (Musca domestica L) femelles adultes anesthésiées de souche SCR. 3 à 5 jours après l'émergence, et par détermination de la mortalité 24 heures après l'application du composé testé, les mouches étant maintenues à 25 °C.

2. Composé suivant la revendication 1, dans lequel R est propyle, butyle, $C_{3-10}$-cycloalcoyle ou phényle et X est $C_{6-10}$-cycloalcoyle, $C_{6-10}$-cycloalcényle, alcynyle ou phényle substitué par un ou plusieurs halo, cyano, azido, nitro ou $C_{1-2}$-alcoyle, l'alcoyle étant substitué par 1 à 3 atomes d'halogène, avec la restriction que R n'est pas phényle lorsque X est 4-fluorophényle.

3. Composé suivant la revendication 2, dans lequel R est propyle, butyle, $C_{5-6}$-cycloalcoyle ou phényle et X est cyclohexyle, cycloheptyle, cyclohexényle, éthynyle ou phényle substitué par halo, cyano, azido, nitro ou $C_{1-2}$-alcoyle, le $C_{1-2}$-alcoyle étant substitué par 1 à 3 atomes d'halogène, avec la restriction que R n'est pas phényle lorsque X est 4-fluorophényle.

4. Composé suivant la revendication 2 ou 3, dans lequel R est n-propyle, butyle, cyclopentyle ou cyclohexyle et X est cyclohexyle, cycloheptyle ou phényle substitué aux positions 3 et/ou 4 par des substituants choisis indépendamment parmi halo, cyano, azido et nitro.

5. Composé suivant la revendication 2 ou 3, dans lequel R est phényle et X est phényle substitué aux positions 3 et/ou 4 par un substituant choisi indépendamment parmi chloro et bromo.

6. Composé suivant la revendication 1, choisi parmi le 1-(4-chlorophényl)-4-n-propyl-2,6,7-trioxabicyclo-[2.2.2]octane, le 1-(4-chlorophényl)-4-n-butyl-2,6,7-trioxabicyclo[2.2.2]octane, le 1-(4-chlorophényl)-4-s-butyl-2,6,7-trioxabicyclo[2.2.2]octane, le 1-(4-chlorophényl)-4-t-butyl-2,6,7-trioxabicyclo[2.2.2]octane, le 1-(4-chlorophényl)-4-cyclopentyl-2,6,7-trioxabicyclo[2.2.2]octane, le 1-(4-chlorophényl)-4-cyclohexyl-2,6,7-trioxabicyclo[2.2.2]-octane, le 1-(4-chlorophényl)-4-phényl-2,6,7-trioxabicyclo[2.2.2]octane, le 1-cyclohexyl-4-t-butyl-2,6,7-trioxabicyclo[2.2.2]octane, le 1-cycloheptyl-4-t-butyl-2,6,7-trioxabicyclo[2.2.2]-octane, le 1-(4-fluorophényl)-4-t-butyl-2,6,7-trioxabicyclo[2.2.2]octane, le 1-(4-bromophényl)-4-t-butyl-2,6,7-trioxabicyclo[2.2.2]octane, le 1-(4-nitrophényl)-4-t-butyl-2,6,7-trioxabicyclo[2.2.2]octane, le 1-(4-cyanophényl)-4-t-butyl-2,6,7-trioxabicyclo[2.2.2]octane, le 1-(3,4-dichlorophényl)-4-t-butyl-2,6,7-trioxabicyclo[2.2.2]octane, le 1-cyclohexyl-4-cyclohexyl-2,6,7-trioxabicyclo[2.2.2]octane, le 1-cycloheptyl-4-cyclohexyl-2,6,7-trioxabicyclo[2.2.2]octane, le 1-(4-fluorophényl)-4-cyclohexyl-2,6,7-trioxabicyclo[2.2.2]octane, le 1-(4-bromophényl)-4-cyclohexyl-2,6,7-trioxabicyclo[2.2.2]octane, le 1-(4-cyanophényl)-4-cyclohexyl-2.6,7-trioxabicyclo[2.2.2]octane et le 1-(3,4-dichlorophényl)-4-cyclohexyl-2,6,7-trioxabicyclo[2.2.2]octane.

7. Composition pesticide comprenant un composé suivant l'une quelconque des revendications 1 à 6 en mélange avec un excipient inerte.

8. Composition pesticide suivant la revendication 7, qui comprend, en outre, un agent de synergie qui inhibe la perte par oxydation du pouvoir toxique du pesticide.

9. Composé suivant l'une quelconque des revendications 1 à 6, pour la lutte contre des organismes nuisibles.

10. Procédé pour lutter contre des organismes nuisibles, qui comprend la mise en contact de ces organismes nuisibles ou de leur environnement avec une quantité efficace pour lutter contre ces organismes nuisibles d'un composé tel que défini dans l'une quelconque des revendications 1 à 6.

11. Procédé pour lutter contre des insectes suivant la revendication 10, dans lequel ces organismes nuisibles ou leur environnement sont également mis en contact avec un agent de synergie qui inhibe la perte par oxydation du pouvoir toxique du pesticide.

12. Procédé de préparation d'un composé de formule :

$R-C(CH_2O)_3C-X$

suivant l'une quelconque des revendications 1 à 6 :

a) à partir d'un triol :

$R-C(CH_2OH)_3$

qui comprend la condensation du triol, catalysée par un acide, avec un orthocarboxylate :

$(R'O)_3C-X$

ou

b) par transposition d'un hydroxyméthyloxétane acylé :

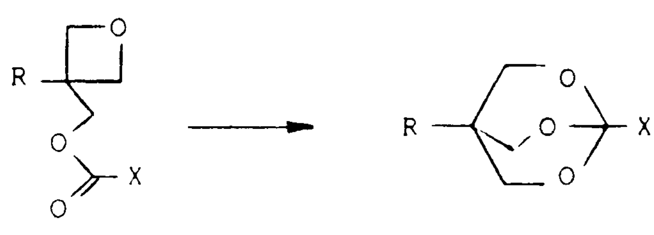

où R et X sont tels que définis à propos de l'une quelconque des revendications 1 à 6.

## Patentansprüche

1. Verbindung R-C($CH_2O$)$_3$C-X, worin R und X jeweils organische Substituenten sind, mit einem pestiziden $LD_{50}$-Wert, ausgedrückt als Mikrogramm der Verbindung pro Gramm Körpergewicht des Schädlings, von nicht mehr als 400, wobei der $LD_{50}$-Wert betimmt wird, indem 0,5 $\mu$l einer Lösung der Verbindung in Aceton oder Tetrahydrofuran topisch dem Ventrum des Abdomens einer anästhetisierten, erwachsenen, weiblichen Hausfliege (Musca domestica L), SCR-Stamm, 3 bis 5 Tage nach dem Befall verabreicht wird und indem die Mortalität 24 Stunden nach der Verabreichung der Testverbindung betimmt wird, wobei die Fliegen bei 25 °C aufbewahrt werden.

2. Verbindung nach Anspruch 1, dadurch **gekennzeichnet,** dass R Propyl, Butyl, $C_{3-10}$ Cycloalkyl oder Phenyl ist und dass X $C_{5-10}$-Cycloalkyl, $C_{6-10}$-Cycloalkenyl, Alkynyl oder Phenyl ist, das durch ein oder mehrere von Halogen, Cyano, Azido, Nitro oder $C_{1-2}$-Alkyl substituiert ist, wobei das Alkyl durch 1 bis 3 Halogenatome substituiert ist, mit dem Vorbehalt, dass R nicht Phenyl ist, wenn X 4-Fluorophenyl ist.

3. Verbindung nach Anspruch 2, dadurch **gekennzeichnet,** dass R Propyl, Butyl, $C_{5-6}$-Cycloalkyl oder Phenyl ist und dass X Cyclohexyl, Cycloheptyl, Cyclohexenyl, Ethynyl oder Phenyl ist, substituiert durch Halogenid, Cyano, Azido, Nitro oder $C_{1-2}$-Alkyl, wobei das $C_{1-2}$-Alkyl durch 1 bis 3 Halogenatome substituiert ist, mit dem Vorbehalt, dass R nicht Phenyl ist, wenn X 4-Fluorophenyl ist.

4. Verbindung nach Anspruch 2 oder 3, dadurch **gekennzeichnet,** dass R n-Propyl, Butyl, Cyclopentyl oder Cyclohexyl ist und dass X Cyclohexyl, Cycloheptyl oder Phenyl ist, das an den 3- und/oder 4-Position(en) durch Substituenten substituiert ist, die unabhängig ausgewählt sind aus Halogenid, Cyano, Azido und Nitro.

5. Verbindung nach einem der Ansprüche 2 oder 3, dadurch **gekennzeichnet,** dass R Phenyl ist und dass X Phenyl ist, das an der 3- und oder 4-Position durch einen Substituenten substituiert ist, der unabhängig aus Chlor und Brom ausgewählt ist.

6. Verbindung nach Anspruch 1, ausgewählt aus 1-(4-Chlorophenyl)-4-n-propyl-2,6,7-trioxabicyclo[2.2.2]-octan, 1-(4-Chlorophenyl)-4-n-butyl-2,6,7-trioxabicyclo[2.2.2]octan, 1-(4-Chlorophenyl)-4-s-butyl-2,6,7-trioxabicyclo[2.2.2]octan, 1-(4-Chlorophenyl)-4-t-butyl-2,6,7-trioxabicyclo[2.2.2]octan, 1-(4-Chlorophenyl)-4-cyclopentyl-2,6,7-trioxabicyclo[2.2.2]octan, 1-(4-Chlorophenyl)-4-cyclohexyl-2,6,7-trioxabicyclo[2.2.2]-octan, 1-(4-Chlorophenyl)-4-phenyl-2,6,7-trioxabicyclo[2.2.2]octan, 1-Cyclohexyl-4-t-butyl-2,6,7-trioxabicyclo[2.2.2]octan, 1-Cycloheptyl-4-t-butyl-2,6,7-trioxabicyclo[2.2.2]octan, 1-(4-Fluorophenyl)-4-t-butyl-2,6,7-trioxabicyclo[2.2.2]octan, 1-(4-Bromophenyl)-4-t-butyl-2,6,7-trioxabicyclo[2.2.2]octan, 1-(4-Nitrophenyl)-4-t-butyl-2,6,7-trioxabicyclo[2.2.2]octan, 1-(4-Cyanophenyl)-4-t-butyl-2,6,7-trioxabicyclo[2.2.2]-octan, 1-(3,4-Dichlorophenyl)-4-t-butyl-2,6,7-trioxabicyclo[2.2.2]octan, 1-Cyclohexyl-4-cyclohexyl-2,6,7-trioxabicyclo[2.2.2]octan, 1-Cycloheptyl-4-cyclohexyl-2,6,7-trioxabicyclo[2.2.2]octan, 1-(4-Fluorophenyl)-4-cyclohexyl-2,6,7-trioxabicyclo[2.2.2]octan, 1-(4-Bromophenyl)-4-cyclohexyl-2,6,7-trioxabicyclo[2.2.2]-octan, 1-(4-Cyanophenyl)-4-cyclohexyl-2,6,7-trioxabicyclo[2.2.2]octan und 1-(3,4-Dichlorophenyl)-4-cyclohexyl-2,6,7-trioxabicyclo[2.2.2]octan.

7. Pestizide Zusammensetzung, umfassend eine Verbindung nach einem der Ansprüche 1 bis 6 in Zumischung mit einem inerten Träger.

8. Pestizide Zusammensetzung nach Anspruch 7, dadurch **gekennzeichnet,** dass sie weiterhin eine synergistische Verbindung enthält, die die oxidative Entgiftung des Pestizides inhibiert.

9. Verbindung nach einem der Ansprüche 1 bis 6 zur Kontrolle von Schädlingen.

10. Verfahren zum Kontrollieren von Schädlingen, umfassend das Kontaktieren der Schädlinge oder deren Umgebung mit einer effektiven Menge einer Verbindung nach einem der Ansprüche 1 bis 6 zum Kontrollieren der Schädlinge.

11. Verfahren zum Kontrollieren von Insekten nach Anspruch 10, dadurch **gekennzeichnet,** dass die Schädlinge oder deren Umgebung mit einem synergistischen Mittel ebenfalls kontaktiert werden, das

die oxidative Entgiftung des Pestizides inhibiert

12. Verfahren zur Herstellung einer Verbindung $R-C(CH_2O)_3C-X$ nach einem der Ansprüche 1 bis 6:
(a) aus einem Triol $R-C(CH_2OH)_3$, umfassend die sauer katalysierte Kondensation des Triols mit einem Orthocarboxylat $(R'O)_3C-X$ oder
(b) durch die Umlagerung eines acylierten Hydroxymethyloxetans

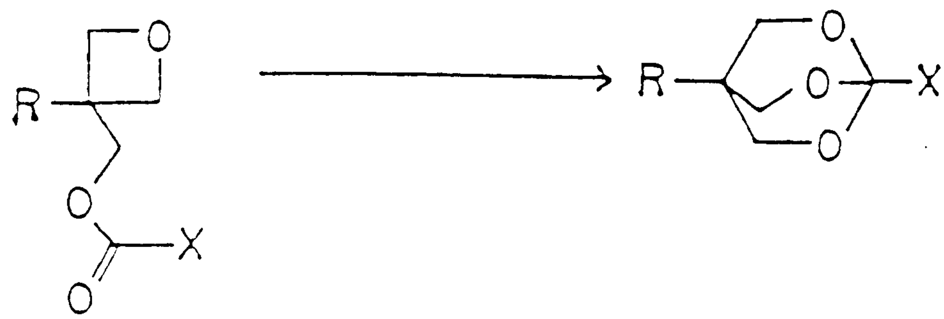

worin R und X die in einem der Ansprüche 1 bis 6 angegebenen Bedeutungen aufweisen.